# EUROPÄISCHE PATENTSCHRIFT

(11) **EP 1 658 874 B1**
(45) Veröffentlichungstag und Bekanntmachung des Hinweises auf die Patenterteilung: **16.07.2014**
(21) Anmeldenummer: 05025335.0
(22) Anmeldetag: 21.11.2005
(51) Int. Cl.: A61M 16/20, F16K 37/00, G01N 33/00, G06K 19/077

(54) **Gasprüfvorrichtung sowie Verfahren zur Prüfung eines über eine Gassteckdose entnehmbaren Gases**
Apparatus and method for checking a gas from a gas socket
Appareil et methode de vérification de gaz extractible à travers un robinet

(30) Priorität: 22.11.2004 DE 102004057394; 15.06.2005 DE 102005027518
(43) Veröffentlichungstag der Anmeldung: 24.05.2006
(73) Patentinhaber: UNIVERSITÄTSKLINIKUM ERLANGEN, 91054 Erlangen (DE)
(72) Erfinder: Stark, Robert, 96114 Hirschaid (DE); Emrich, Wolfgang, 91325 Adelsdorf (DE)
(74) Vertreter: Gassner, Wolfgang

(56) Entgegenhaltungen:
- EP-A2- 1 441 222
- GB-A- 2 328 839
- US-A- 5 356 594
- US-A1- 2002 014 266
- US-B1- 6 266 995

## Beschreibung

Die Erfindung betrifft eine Gasprüfvorrichtung zum Prüfen eines über eine Gassteckdose entnehmbaren Gases sowie ein Verfahren zur Prüfung eines über eine Gassteckdose entnehmbaren Gases. Die Erfindung betrifft insbesondere das Gebiet von Gasversorgungseinrichtungen in Krankenhäusern und deren Prüfung.

Nach dem Europäischen Arzneimittelbuch, AMG, MPG, EN 737, DIN 1946 ist es erforderlich, Gasversorgungseinrichtungen zur Bereitstellung medizinischer Gase in Krankenhäusern regelmäßig zu prüfen. Derartige Gasversorgungseinrichtungen umfassen in der Regel eine Vielzahl von Gasarmaturen, insbesondere Gassteckdosen. Nach den gültigen gesetzlichen Vorgaben ist jede Gassteckdose zu prüfen. Die dabei gewonnenen Messergebnisse sind zu dokumentieren und zu archivieren.

Nach dem Stand der Technik bedient man sich zu diesem Zweck mobiler Prüfvorrichtungen, mit denen je nach Art des zu prüfenden Gases die vorgeschriebenen Prüfungen durchgeführt werden. Dabei werden auf Messeinrichtungen angezeigte Werte visuell abgelesen und in Tabellen übertragen. - Es ist offensichtlich, dass das bekannte Verfahren zeitaufwändig und fehleranfällig ist. Es kann insbesondere beim Ablesen von Messwerten zu Fehlern kommen. Abgesehen davon können weitere Fehler dadurch entstehen, dass eine Gassteckdose nicht korrekt identifiziert wird oder ein abgelesener Wert in eine falsche Tabelle übertragen wird. Wegen des enormen Zeitaufwands der Prüfung von üblicherweise mehreren tausend Gassteckdosen in einem Krankenhaus können z. B. eine eventuelle Verunreinigung eines Gases in einem Leitungsast nur mit einer Zeitverzögerung erkannt und entsprechende Gegenmaßnahmen eingeleitet werden.

Die US 6 266 995 B1 offenbart eine Gasprüfvorrichtung und ein Verfahren zur Prüfung für Gasversorgungsysteme im medizinischen Umfeld. Sie umfasst eine Messeinrichtung und einen der Datenverarbeitung und -speicherung dienenden Computer mit einer Vielfalt an Benutzerschnittstellen. Nach dieser Druckschrift muss der Benutzer aller Informationen (insbesondere Montagenort und Gasart) manuell eingeben.

Die US 2002/0142661 A1 vorschlägt die Verwendung von Identifikationsmitteln wie Barcodes bei Gasflussregler auf dem medizinischen Gebiet.

Die EP 1 441 222 A2 offenbart eine Vorrichtung zum Überprüfen des über eine mechanische Atemhilfe einem Patienten verabreichten Gases während des normalen Betriebs.

Aufgabe der Erfindung ist es, die Nachteile nach dem Stand der Technik zu beseitigen. Es sollen insbesondere eine Gasprüfvorrichtung sowie ein Verfahren zur Prüfung eines über eine Gassteckdose entnehmbaren Gases angegeben werden, welche eine zeitsparende und exakte Prüfung von Gasen ermöglichen. Nach einem weiteren Ziel der Erfindung sollen Fehler bei der Messung, Dokumentation und Archivierung vermindert bzw. völlig ausgeschlossen werden.

Diese Aufgabe wird durch die Merkmale der Ansprüche 1 und 9 gelöst. Zweckmäßige Ausgestaltungen ergeben sich aus den Merkmalen der Ansprüche 2 bis 8 und 10 - 19.

Zur Verwendung in der Erfindung wird eine Gassteckdose mit einem daran vorgesehenen maschinenlesbaren Identifikationsmittel vorgeschlagen. - Wegen der vorgeschlagenen Maschinenleabarkeit des Identifikationsmittels können Fehler bei der Informationsübermittlung vermieden werden. Die an der Gassteckdose bei der Durchführung eines Prüfungsverfahrens gewonnenen Messwerte können zwangsläufig und eindeutig mit einer aus dem Identifikationsmittel ausgelesenen Informationen korreliert und in einem gemeinsamen Datensatz abgespeichert werden. Das ermöglicht eine eindeutige und fehlerfreie Dokumentation von Messwerten.

Nach einer vorteilhaften Ausgestaltung ist die im Identifikationsmittel enthaltene Information kontaktlos, vorzugsweise optisch oder elektromagnetisch, auslesbar. Es kann aber auch sein, dass die in dem Identifikationsmittel enthaltene Information durch einen mechanischen Kontakt, vorzugsweise mittels Berührungssensoren, auslesbar ist. Das Identifikationsmittel kann insbesondere ein RFID-Transponder, ein Barcode oder ein mechanisch erfassbares Relief sein. Es ist insbesondere so auageführt, dass damit auch mehrere Informationen in kodierter Form gespeichert werden können. Das Identifikationsmittel kann insbesondere eine oder mehrere der folgenden Informationen enthalten: Art des an der Gassteckdose angeschlossenen Gases, Montageort der Gassteckdose, maximaler und/oder minimaler Solldruck. Wie aus der vorstehenden Aufzählung ersichtlich ist, kann das Identifikationsmittel insbesondere auch Informationen enthalten, welche zur Einstellung von Parametern einer daran anzuschließenden Gasprüfvorrichtung dienen. Das Identifikationsmittel kann insbesondere auch alle für die jeweilige Gassteckdose relevanten Parameter oder Parameterbereiche als Information enthalten. Diese können an eine Gasprüfvorrichtung übergeben werden und es kann damit geprüft werden, ob ein an der Gassteckdose anstehendes Gas in seinen Eigenschaften den Parametern entspricht.

Nach einer besonders vorteilhaften Ausgestaltung der Erfindung ist vorgesehen, dass das Identifikationsmittel im Bereich einer eine Einstecköffnung aufweisenden Frontseite der Gassteckdose vorgesehen ist. Eine solche Einstecköffnung dient dem Anschluss eines Gasschlauchs einer Gasprüfvorrichtung. Beim Vorsehen des Identifikationsmittels im Bereich der Einstecköffnung kann eine zum Auslesen des Identifikationsmittels geeignete Erfassungseinrichtung zweckmäßigerweise im Bereich eines Anschlussstutzens eines in die Einstecköffnung einzusteckenden Prüfschlauchs angebracht werden.

Nach weiterer Maßgabe der Erfindung ist eine Gasprüfvorrichtung zum Prüfen eines über die Gassteckdose entnehmbaren Gases vorgesehen mit einer Erfassungseinrichtung zum Erfassen der im Identifikationsmittel enthaltenen Information und einem Mittel zum Erzeugen eines die Information und die nachfolgend an der Gassteckdose gemessene Werte enthaltenden Datensatzes. - Die erfindungsgemäß vorgeschlagene Gasprüfvorrichtung wirkt mit der vorstehend beschriebenen Gassteckdose vorteilhafterweise derart zusammen, dass damit bei einer Prüfung des an der Gasarmatur anstehenden Gases gewonnene Messwerte eindeutig mit einer aus dem Identifikationsmittel automatisch ausgelesenen Information korreliert und zu einem gemeinsamen Datensatz verarbeitet werden können. Damit wird eine fehlerhafte Zuordnung gewonnener Messwerte praktisch ausgeschlossen. Es wird eine praktisch fehlerfreie Dokumentation der Messwerte ermöglicht.

Die Erfassungseinrichtung kann einen Barcode-Scanner, eine Einrichtung zum Empfangen zumindest einer in einem RFID-Transponder gespeicherten Information oder eine Abtasteinrichtung zum Abtasten eines Reliefs umfassen. Je nach verwendetem Identifikationsmittel können auch andere Erfassungseinrichtungen vorgesehen sein. Die Erfassungseinrichtung kann ferner eine Einrichtung zur Auswertung der kodierten Information, beispielsweise deren Umwandlung in Parameter oder einen Text, umfassen.

Darüber hinaus umfasst die Gasprüfvorrichtung eine Messeinrichtung zum Messen zumindest eines Gasparameters und einen mit der Messeinrichtung verbundenen Gasschlauch zum Verbinden der Messeinrichtung mit der Gasarmatur. Nach einer besonders vorteilhaften Ausgestaltung ist die Erfassungseinrichtung im Bereich eines Anschlussstutzens des Gasschlauchs angebracht, so dass bei einem Anschluss des Anschlussstutzens an der Gasarmatur die Information auslesbar ist. Insbesondere kann die Anbringung der Erfassungseinrichtung im Bereich des Anschlussstutzens so ausgestaltet sein, dass nur bei einem korrekten Anschluss des Anschlussstutzens an der Gasarmatur ein Auslesen der Information mittels der Erfassungseinrichtung möglich ist. Damit kann die Durchführung einer Prüfroutine von vornherein unterbunden werden, wenn kein korrekter Anschluss des Anschlussstutzens an der Gasarmatur hergestellt worden ist.

Nach einer weiteren Ausgestaltung der Erfindung ist vorgesehen, dass die Messeinrichtung ein Messmittel zur Messung bzw. Bestimmung zumindest eines der folgenden Parameter umfasst: Druck, vorzugsweise statischer und/oder dynamischer Druck, Temperatur, Volumenstrom, Massenstrom, Gasart, Taupunkt, Feuchtigkeitsgehalt, O₂-Gehalt, CO₂-Gehalt, N₂O-Gehalt. Das Messmittel kann zur Messung der vorgenannten Parameter geeignete Sensoren umfassen. Die Sensoren können im Hinblick auf die jeweils zu messende Gasart zu dafür spezifischen Messstrecken zusammengefasst sein. Es ist aber auch möglich, bestimmte Sensoren, beispielsweise Sensoren zur Messung des Drucks oder dgl., gemeinsam für jede der zu prüfenden Gasarten zu verwenden.

Erfindungsgemäß ist auch eine Auswahlvorrichtung zum Auswählen eines von mehreren vorgegebenen Prüfverfahren in Abhängigkeit der erfassten Information und/oder der ermittelten Gasart vorgesehen. Eine Information über die prüfende Gasart kann also im Identifikationsmittel gespeichert und an die Gasprüfvorrichtung übermittelt werden. Infolgedessen kann automatisch ein für die jeweilige Gasart geeignetes Prüfverfahren aus mehreren vorgegebenen Prüfverfahren ausgewählt werden. Das Prüfverfahren kann anschließend, z. B. nach Freigabe durch einen Benutzer, gestartet werden. Die Auswahl eines geeigneten Prüfverfahrens kann aber auch in Abhängigkeit einer mittels der Messeinrichtung bestimmten Gasart erfolgen.

Es wird auch eine Plausibilitätsprüfung vorgenommen, indem z. B. die im Identifikationsmittel enthaltene Information über die Gasart und das Ergebnis der Bestimmung der Gasart mittels der Messeinrichtung verglichen werden. Damit kann auf einfache und zuverlässige Weise festgestellt werden, ob beispielsweise an einer Gasarmatur eine unzulässige Gasart ansteht.

Nach einer weiteren Ausgestaltung der Erfindung ist ein Anzeigemittel, vorzugsweise ein Flachbildschirm, zur Anzeige der Messwerte vorgesehen. Der Flachbildschirm kann insbesondere Bestandteil eines tragbaren Computers sein, der mit einem Programm zur Erfassung und Auswertung der von der Messeinrichtung gelieferten Messwerte versehen ist. Zur Bedienung eines solchen tragbaren Computers ist zweckmäßigerweise eine Folientastatur vorgesehen, welche die im Klinikbereich gültigen Hygienevorschriften erfüllt, d. h. sie weist insbesondere keine Ritzen und Spalten auf, in denen mit Keimen beladener Schmutz sich ansammeln kann.

Desgleichen können die übrigen Komponenten der Gasprüfvorrichtung in einem entsprechend gestalteten Gehäuse untergebracht sein, welches eine die Anlagerung von Keimen abweisende Oberfläche aufweist. Eine solche Oberfläche ist möglichst glatt ausgebildet. Sie kann beispielsweise mit einem Kunststoff beschichtet sein, der antimikrobielle Eigenschaften hat. Ein solcher Kunststoff kann beispielsweise mit Silberpartikeln versehen sein.

Die Gasprüfvorrichtung kann des Weiteren ein Mittel zum Speichern des Datensatzes umfassen. Sie kann ferner eine Schnittstelle zur Übertragung des gespeicherten Datensatzes an eine Datenverarbeitungseinrichtung umfassen. Bei der Schnittstelle kann es sich um eine Schnittstelle zur kabellosen Übertragung des Datensatzes handeln. Mit den vorgeschlagenen Merkmalen ist es möglich, die vorzugsweise mobil ausgestaltete Gasprüfvorrichtung zur Prüfung einer Vielzahl Gassteckdosen von einer Gassteckdose zur nächsten zu bewegen und am Ende eines Prüfdurchgangs die gesammelten Datensätze zur Archivierung an eine Datenverarbeitungseinrichtung zu übertragen.

Um eine besonders hohe Mobilität der Gasprüfvorrichtung zu gewährleisten, ist nach einer weiteren Ausgestaltung eine netzunabhängige Stromversorgungseinrichtung vorgesehen. Es kann sich dabei um einen Akku handeln.

Nach weiterer Maßgabe der Erfindung ist ein Verfahren zur Prüfung eines über entnehmbaren Gases vorgesehen gemäß Anspruch 9.

Wegen der vorteilhaften Ausgestaltung des Verfahrens wird auf die vorangegangenen Ausführungen zu den vorteilhaften Ausgestaltungen der Gasprüfvorrichtung verwiesen, die sinngemäß auch für das Verfahren anwendbar sind.

Im Sinne der vorliegenden Erfindung wird unter einem "Gas" auch Druckluft oder Vakuum verstanden. Das Gas im Sinne der vorliegenden Erfindung umfasst insbesondere medizinische Gase, wie O₂, N₂O und dgl..

Nachfolgend wird ein Ausführungsbeispiel der Erfindung anhand der Zeichnungen näher erläutert. Es zeigen:
- Fig. 1: eine schematische Darstellung eines Systems zur Prüfung und
- Fig. 2: ein Flussdiagramm.

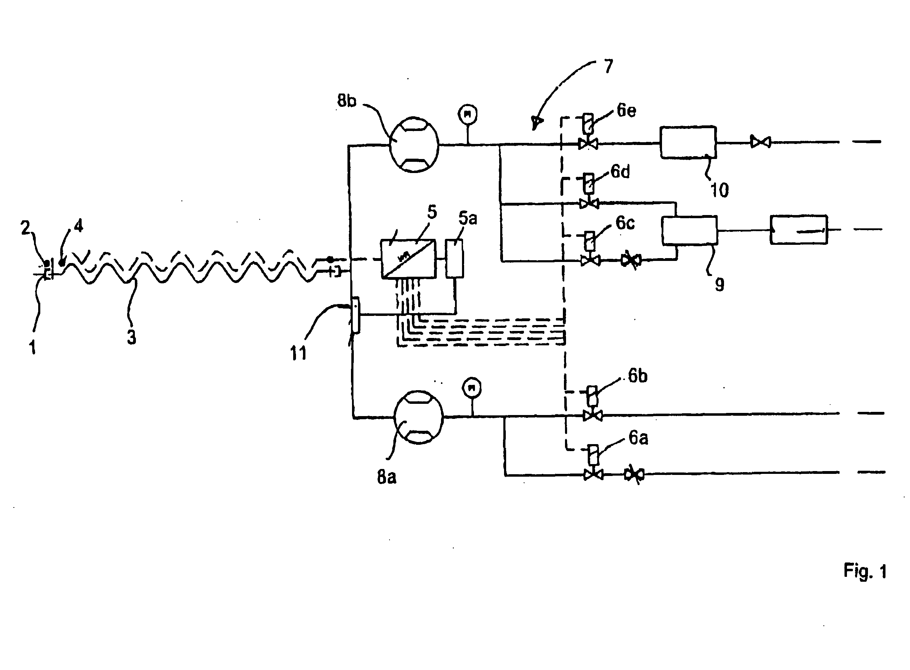

In Fig. 1 ist eine Gasarmatur 1, insbesondere eine Gassteckdose, mit einem maschinenlesbaren Identifikationsmittel 2 versehen. Es kann sich dabei insbesondere um einen optisch auslesbaren Barcode oder auch einen RFID-Transponder, handeln. An der Gasarmatur 1 ist ein (hier nicht gesondert dargestellter) Anschlussstutzen eines Gasschlauchs 3 angeschlossen. Bei ordnungsgemäß angeschlossenem Anschlussstutzen befindet sich eine, z. B. am Anschlussstutzen angebrachte, Erfassungseinrichtung 4 in der Nähe des Identifikationsmittels 2, so dass eine im Identifikationsmittel 2 gespeicherte Information mit der Erfassungseinrichtung 4 auslesbar ist. Bei der Erfassungseinrichtung 4 kann es sich um einen Barcode-Scanner, eine Einrichtung zur Erfassung von in RFID-Transpondern gespeicherten Informationen oder dgl. handeln.

Zweckmäßigerweise sind im Identifikationsmittel 2 eine oder mehrere der folgenden Informationen in kodierter Form gespeichert:
- Information zur eindeutigen Identifizierung der Gasarmatur 1, insbesondere eines Montageorts;
- Information über die an der Gasarmatur 1 anstehende Gasart;
- Informationen über vorgegebene Gasparameter, z. B. Druck, Feuchtigkeit, O₂-Gehalt und dgl..

Die Erfassungseinrichtung 4 ist mit einem Computer 5 verbunden, welche eine Speichereinheit 5a umfasst. Der Computer 5 umfasst ein Programm, mit dem Ventile 6a, ..., 6e in einer allgemein mit dem Bezugszeichen 7 bezeichneten Messeinrichtung steuerbar sind.

Die Messeinrichtung 7 umfasst ferner erste Sensoren 8a, 8b zur Erfassung des Masse- und/oder Volumenstroms, einen zweiten Sensor 9 zur Bestimmung des CO₂-Gehalts und einen dritten Sensor 10 zur Ermittlung des Feuchtigkeitsgehalts. Daneben können - je nach Bedarf - auch andere Sensoren vorgesehen sein. Mit dem Bezugszeichen 11 ist eine Schnittstelle, z. B. eine USB-, Bluetooth- oder RS232-Schnittstelle, bezeichnet.

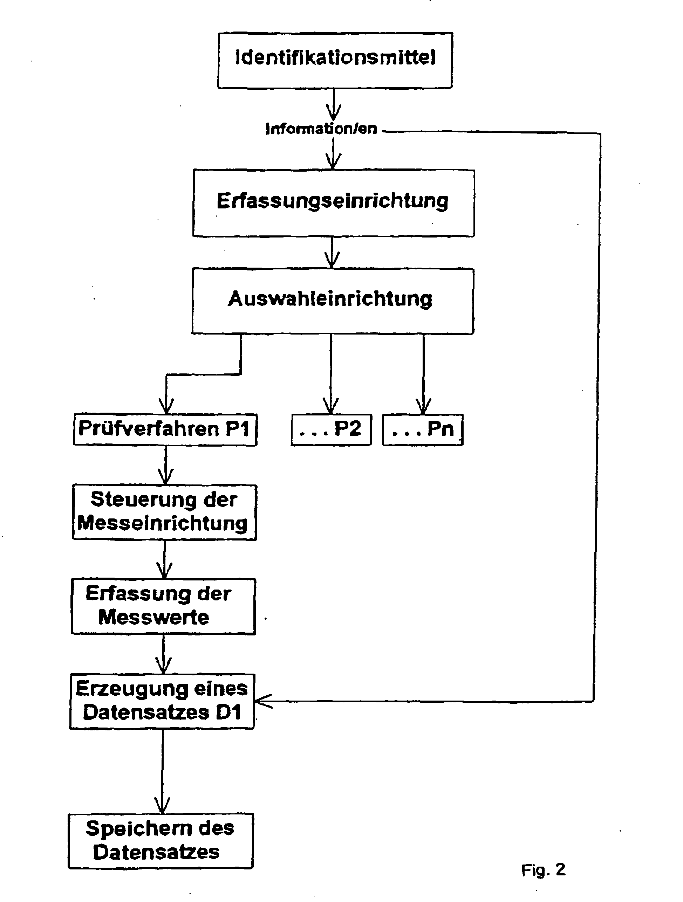

Das mit der Vorrichtung durchführbare Verfahren wird anhand des in Fig. 2 gezeigten Flussdiagramms näher erläutert.

Beim Anschluss des Gasschlauchs 3 an der Gasarmatur 1 wird mittels der Erfassungseinrichtung 4 automatisch die im Identifikationsmittel 2 gespeicherte Information ausgelesen und an den Computer 5 übermittelt. Auf der Grundlage der Information wird mit einem im Computer 5 gespeicherten Programm automatisch aus einer Mehrzahl von Prüfverfahren P1, ..., Pn ein für das an der Gasarmatur 1 anstehende Gas geeignetes Prüfungsverfahren P1 ausgewählt und, beispielsweise über einen Flachbildschirm, dem Benutzer angezeigt. Die Auswahl kann beispielsweise auf der Grundlage einer Information erfolgen, welche die anstehende Gasart oder die spezifische Kennung der Gasarmatur 1 betrifft. Im letzteren Fall kann beispielsweise in einer Tabelle zu jeder zu prüfenden Gasarmatur 1 ein Prüfverfahren hinterlegt sein und das Prüfverfahren unter Verwendung dieser Tabelle ausgewählt werden.

Nach einem benutzerseitig ausgewählten Start des Prüfverfahrens P1 werden die Ventile 6a, ..., 6e der Messeinrichtung 7 mittels des Programms in geeigneter Weise so gesteuert, dass die Sensoren 8a, 8b, 9, 10 in geeigneter Weise mit Gas beaufschlagt werden. Dazu können die Ventile 6a, ..., 6e für im Programm hinterlegte vorgegebene Zeiten geöffnet und nachfolgend wieder geschlossen werden. Die Steuerung der Ventile 6a, ..., 6e kann zusätzlich in Abhängigkeit von mittels der Sensoren 8a, 8b gemessener Werte erfolgen. Die mit den Sensoren 8a, 8b, 9, 10 gemessenen Werte werden an den Computer 5 übertragen und zusammen mit zumindest einer die Gasarmatur 1 identifizierenden Information, welche beispielsweise der Montageort sein kann, zu einem Datensatz D1 verarbeitet und in der Speichereinheit 5a gespeichert.

Gleichzeitig können auf einer Anzeigevorrichtung dem Benutzer die gemessenen Werte angezeigt und ggf. ein Warnhinweis ausgegebenen werden, falls diese nicht in einem vorgegebenen Parameterbereich liegen.

Im gespeicherten Datensatz D1 sind die jeweiligen Messwerte in eindeutiger Zuordnung zu jeder geprüften Gasarmatur 1 unter dem jeweiligen Prüfdatum gespeichert. Das Programm kann insbesondere so ausgelegt sein, dass eine Manipulation der gemessenen Werte unmöglich ist. Der Datensatz D1 kann zu einem späteren Zeitpunkt zu Archivierungszwecken beispielsweise über die Schnittstelle 11 oder auch andere geeignete Übertragungsmittel an eine Datenverarbeitungseinrichtung (hier nicht gezeigt) übermittelt werden.

### Bezugszeichenliste

- 1: Gasarmatur
- 2: Identifikationsmittel
- 3: Gasschlauch
- 4: Erfassungseinrichtung
- 5: Computer
- 5a: Speichereinheit
- 6a, ..., 6e: Ventil
- 7: Messeinrichtung
- 8a, 8b: erster Sensor
- 9: zweiter Sensor
- 10: dritter Sensor
- 11: Schnittstelle
- D1: Datensatz
- P1, ..., Pn: Prüfverfahren

## Patentansprüche

1. Gasprüfvorrichtung zum Prüfen eines über eine Gassteckdose mit einem daran vorgesehenen maschinenlesbaren Identifikationsmittel (2) entnehmbaren Gases mit
einer Erfassungseinrichtung (4) zum Erfassen einer im Identifikationsmittel (2) enthaltenen Information über die Art einer an der Gassteckdose angeschlossenen Soll-Gasart,
einer Auswahlvorrichtung (5) zum automatischen Auswählen eines von mehreren vorgegebenen Prüfverfahren (P1, ..., Pn) in Abhängigkeit der erfassten Information,
einer Messeinrichtung (7) zum Messen zumindest eines Gasparameters und einem mit der Messeinrichtung (7) verbundenen Gasschlauch (3) zum Verbinden der Messeinrichtung (7) mit der Gassteckdose,
einer Plausibilitätsprüfeinrichtung zum Vergleichen der über das Identifikationsmittel erhaltenen Information über die Soll-Gasart und der mit der Messeinrichtung (7) ermittelten Ist-Gasart, und
einem Mittel zum Erzeugen eines die Information und den zumindest einen gemessenen Gasparameter enthaltenden Datensatzes (D1).

2. Gasprüfvorrichtung nach Anspruch 1, wobei die Erfassungseinrichtung (4) einen Barcode-Scanner, eine Einrichtung zum Empfangen zumindest einer in einem RFID-Transponder gespeicherten Information oder eine Abtasteinrichtung zum Abtasten eines Reliefs umfasst.

3. Gasprüfvorrichtung nach einem der Ansprüche 1 oder 2, wobei die Erfassungseinrichtung (4) im Bereich eines Anschlussstutzens des Gasschlauchs (3) angebracht ist, so dass bei einem Anschluss des Anschlussstutzens an der Gassteckdose die Information auslesbar ist.

4. Gasprüfvorrichtung nach einem der vorhergehenden Ansprüche, wobei die Messeinrichtung (7) ein Messmittel zur Messung (8a, 8b, 9, 10) bzw. Bestimmung zumindest eines der folgenden Gasparameter umfasst: Druck, vorzugsweise statischer und/oder dynamischer Druck, Temperatur, Volumenstrom, Massenstrom, Taupunkt, Feuchtigkeitsgehalt, O₂-Gehalt, CO₂-Gehalt, N₂O-Gehalt.

5. Gasprüfvorrichtung nach einem der vorhergehenden Ansprüche, wobei ein Anzeigemittel, vorzugsweise ein Flachbildschirm, zur Anzeige der gemessenen Gasparameter vorgesehen ist.

6. Gasprüfvorrichtung nach einem der vorhergehenden Ansprüche, wobei ein Mittel (5a) zum Speichern des Datensatzes (D1) vorgesehen ist.

7. Gasprüfvorrichtung nach einem der vorhergehenden Ansprüche, wobei eine Schnittstelle (11) zur Übertragung des gespeicherten Datensatzes (D1) an eine Datenverarbeitungseinrichtung vorgesehen ist.

8. Gasprüfvorrichtung nach einem der vorhergehenden Ansprüche, wobei die Schnittstelle (11) eine Schnittstelle zur kabellosen Übertragung des Datensatzes (D1) ist.

9. Verfahren zur Prüfung eines über eine Gassteckdose entnehmbaren Gases mit folgenden Schritten:
Bereitstellen eines an der Gassteckdose vorgesehenen maschinenlesbaren Identifikationsmittels (2),
Erfassen zumindest einer im Identifikationsmittel (2) enthaltenen Information über die Art einer an der Gassteckdose angeschlossenen Soll-Gasart mittels einer an einer Gasprüfvorrichtung vorgesehenen Erfassungseinrichtung (4),
Übermittlung der ausgelesenen Information an eine Auswahlvorrichtung (5) und automatische Auswahl eines Prüfverfahrens aus einer Gruppe von mehreren vorgegebenen Prüfverfahren (P1, ..., Pn) in Abhängigkeit der Information,
Messen zumindest eines Gasparameters mittels einer in der Gasprüfvorrichtung vorgesehenen Messeinrichtung (7),
Vergleichen der über das Identifikationsmittel erhaltenen Information über die Soll-Gasart und der mit der Messeinrichtung (7) ermittelten Ist-Gasart, und
Erzeugen eines die Information und den zumindest einen Gasparameter enthaltenden Datensatzes (D1).

10. Verfahren nach Anspruch 9, wobei die im Identifikationsmittel (2) enthaltene Information kontaktlos, vorzugsweise optisch oder elektromagnetisch, ausgelesen wird.

11. Verfahren nach einem der Ansprüche 9 oder 10, wobei die im Identifikationsmittel (2) enthaltene Information durch einen mechanischen Kontakt, vorzugsweise mittels Berührungssensoren, ausgelesen wird.

12. Verfahren nach einem der Ansprüche 9 bis 11, wobei als Identifikationsmittel (2) ein RFID Transponder, ein Barcode oder ein mechanisch erfassbares Relief verwendet wird.

13. Verfahren nach einem der Ansprüche 9 bis 12, wobei eine oder mehrere der folgenden Informationen von der Gasprüfvorrichtung aus dem Identifikationsmittel (2) ausgelesen werden: Montageort der Gassteckdose, maximaler und/oder minimaler Solldruck.

14. Verfahren nach einem der Ansprüche 9 bis 13, wobei die Information mittels eines Barcode-Scanners, einer Einrichtung zum Empfangen von in einem RFID-Transponder gespeicherten Informationen, oder einer Abtasteinrichtung zum Abtasten eines Reliefs erfasst wird.

15. Verfahren nach einem der Ansprüche 9 bis 14, wobei die Erfassungseinrichtung (4) im Bereich eines Anschlussstutzens des Gasschlauchs (3) angebracht ist, so dass erst bei einem Anschluss des Anschlussstutzens an der Gassteckdose die Information ausgelesen wird.

16. Verfahren nach einem der Ansprüche 9 bis 15, wobei mittels der Messeinrichtung (7) zumindest einer der folgenden Gasparameter gemessen wird: Druck, vorzugsweise statischer Druck und/oder dynamischer Druck, Temperatur, Volumenstrom, Massenstrom, Taupunkt, Feuchtigkeitsgehalt, O₂-Gehalt, CO₂-Gehalt, N₂O-Gehalt.

17. Verfahren nach einem der Ansprüche 9 bis 16, wobei die gemessenen Gasparameter mittels eines Anzeigemittels, vorzugsweise einem Flachbildschirm, angezeigt werden.

18. Verfahren nach einem der Ansprüche 9 bis 17, wobei die gemessenen Gasparameter in einem Mittel (5a) zum Speichern gespeichert werden.

19. Verfahren nach einem der Ansprüche 9 bis 18, wobei der Datensatz (D1) über eine Schnittstelle (11) an eine Datenverarbeitungseinrichtung übertragen wird.

## Claims

1. A gas testing apparatus for testing a gas that can be taken from a gas socket with a machine-readable identification means provided thereon, comprising
a detection device (4) for detecting a piece of information, contained in the identification means (2), concerning the type of a target gas type connected to the gas socket,
a selecting apparatus (5) for automatically selecting one of a number of predefined test methods (P1, ..., Pn) in accordance with the detected information,
a measuring device (7) for measuring a gas parameter, and a gas tube connected to the measuring device (7) for connecting the measuring device (7) to the gas socket,
a plausibility testing device for comparing the information, concerning the target gas type, obtained via the identification means and the actual gas type determined by means of the measuring device (7), and
a means for generating a dataset (D1) containing the at least one measured gas parameter.

2. The gas testing apparatus according to Claim 1, wherein the detection device (4) comprises a barcode scanner, a device for receiving at least one piece of information stored in an RFID transponder, or a scanning device for scanning a relief.

3. The gas testing apparatus according to one of Claims 1 or 2, wherein the detection device (4) is fitted in the region of a connection piece of the gas tube (3), such that the information can be read out when the connection tube is attached to the gas socket.

4. The gas testing apparatus according to one of the preceding claims, wherein the measuring device (7) comprises a measurement means for measuring (8a, 8b, 9, 10) or determining at least one of the following gas parameters: pressure, preferably static and/or dynamic pressure, temperature, volume flow rate, mass flow rate, dew point, moisture content, O₂ content, CO₂ content, N₂O content.

5. The gas testing apparatus according to one of the preceding claims, wherein a display means, preferably a flat screen, is provided for displaying the measured gas parameter(s).

6. The gas testing apparatus according to one of the preceding claims, wherein a means (5a) is provided for storing the dataset (D1).

7. The gas testing apparatus according to one of the preceding claims, wherein an interface (11) is provided for transmitting the stored dataset (D1) to a data processing device.

8. The gas testing apparatus according to one of the preceding claims, wherein the interface (11) is an interface for wireless transmission of the dataset (D1).

9. A method for testing a gas that can be taken from a gas socket comprising the following steps:
providing a machine-readable identification means (2) on the gas socket,
detecting at least one piece of information, contained in the identification means (2), concerning the type of a target gas type connected to the gas socket by means of a detection device (4) provided on a gas testing apparatus,
transmitting the read-out information to a selecting apparatus (5) and automatically selecting a test method from a group of a number of predefined test methods (P1, ..., Pn) in accordance with the detected information,
measuring a gas parameter by means of a measuring device (7) provided in the gas testing apparatus,
comparing the information, concerning the target gas type, obtained via the identification means and the actual gas type determined by means of the measuring device (7), and
generating a dataset (D1) containing the information and the at least one gas parameter.

10. The method according to Claim 9, wherein the information contained in the identification means (2) is read out contactlessly, preferably optically or electromagnetically.

11. The method according to one of Claims 9 or 10, wherein the information contained in the identification means (2) is read out by a mechanical contact, preferably by means of contact sensors.

12. The method according to one of Claims 9 to 11, wherein an RFID transponder, a barcode or a mechanically detectable relief is used as an identification means (2).

13. The method according to one of Claims 9 to 12, wherein one or more of the following pieces of information is/are read out by the gas testing apparatus from the identification means (2): installation location of the gas socket, maximum and/or minimum target pressure.

14. The method according to one of Claims 9 to 13, wherein the information is detected by means of a barcode scanner, a device for receiving information stored in an RFID transponder, or a scanning device for scanning a relief.

15. The method according to one of Clams 9 to 14, wherein the detection device (4) is fitted in the region of a connection piece of the gas tube (3), such that the information is only read out once the connection piece is attached to the gas socket.

16. The method according to one of Claims 9 to 15, wherein at least one of the following gas parameters is/are measured by means of the measuring device (7): pressure, preferably static pressure and/or dynamic pressure, temperature, volume flow rate, mass flow rate, dew point, moisture content, O₂ content, CO₂ content, N₂O content.

17. The method according to one of Claims 9 to 16, wherein the measured gas parameter(s) is/are displayed by means of a display means, preferably a flat screen.

18. The method according to one of Claims 9 to 17, wherein the measured gas parameter(s) is/are stored in a means (5a) for storing.

19. The method according to one of Claims 9 to 18, wherein the dataset (D1) is transmitted via an interface (11) to a data processing device.

## Revendications

1. Système de contrôle de gaz permettant de contrôler un gaz prélevable par l'intermédiaire d'une prise de gaz avec un moyen d'identification (2) lisible par machine prévu sur cette dernière, comportant
un dispositif de saisie (4) permettant de saisir une information contenue dans le moyen d'identification (2) relative à la nature d'un gaz consigne raccordé à la prise de gaz,
un système de sélection (5) permettant de sélectionner automatiquement l'une de plusieurs méthodes de contrôle prédéfinies (P1, ..., Pn) en fonction de l'information saisie,
un dispositif de mesure (7) permettant de mesurer au moins un paramètre de gaz et avec un tuyau de gaz (3) raccordé au dispositif de mesure (7) pour relier le dispositif de mesure (7) à la prise de gaz,
un dispositif de contrôle de vraisemblance permettant de comparer l'information contenue sur la nature de gaz consigne par l'intermédiaire du moyen d'identification et la nature de gaz réel déterminée avec le dispositif de mesure (7), et
un moyen permettant de générer un enregistrement de données (D1) contenant l'information et au moins l'un d'un paramètre de gaz mesuré.

2. Système de contrôle de gaz selon la revendication 1, selon lequel le dispositif de saisie (4) comporte un lecteur de code-barres, un appareil pour la réception d'au moins une information enregistrée dans un transpondeur RFID ou un dispositif de balayage pour balayer un relief.

3. Système de contrôle de gaz selon l'une des revendications 1 ou 2, selon lequel le dispositif de saisie (4) est monté dans la zone d'un raccord du tuyau de gaz (3), de sorte que l'information soit lisible dans le cas d'un raccordement du raccord à la prise de gaz.

4. Système de contrôle de gaz selon l'une des revendications précédentes, selon lequel le dispositif de mesure (7) comporte un moyen de mesure permettant la mesure (8a, 8b, 9, 10) ou la détermination d'au moins l'un des paramètres de gaz suivants: pression, de préférence pression statique et/ou dynamique, température, débit volumique, débit massique, point de rosée, teneur en humidité, teneur en O₂, teneur en CO₂, teneur en N₂O.

5. Système de contrôle de gaz selon l'une des revendications précédentes, selon lequel un moyen d'affichage, de préférence un écran plat, est prévu pour l'affichage des paramètres de gaz mesurés.

6. Système de contrôle de gaz selon l'une des revendications précédentes, selon lequel un moyen (5a) est prévu pour la mémorisation de l'enregistrement de données (D1).

7. Système de contrôle de gaz selon l'une des revendications précédentes, selon lequel une interface (11) est prévue pour la transmission de l'enregistrement de données mémorisé (D1) à un dispositif de traitement des données.

8. Système de contrôle de gaz selon l'une des revendications précédentes, selon lequel l'interface (11) est une interface servant à la transmission sans fil de l'enregistrement de données (D1).

9. Méthode de contrôle d'un gaz prélevable par l'intermédiaire d'une prise de gaz selon les opérations suivantes:
mise à disposition d'un moyen d'identification (2) lisible par machine prévu sur la prise de gaz,
saisie d'au moins une information contenue dans le moyen d'identification (2) sur la nature d'un gaz consigne raccordé à la prise de gaz au moyen d'un dispositif de saisie (4) prévu sur un système de contrôle de gaz,
transmission de l'information lue à un système de sélection (5) et sélection automatique d'une méthode de contrôle à partir d'un groupe de plusieurs méthodes de contrôle prédéfinies (P1, ..., Pn) en fonction de l'information,
mesure d'au moins un paramètre de gaz au moyen d'un dispositif de mesure (7) prévu dans le système de contrôle de gaz,
comparaison de l'information contenue par l'intermédiaire du moyen d'identification sur la nature de gaz consigne et de la nature de gaz réel déterminée avec le dispositif de mesure (7), et
génération d'un enregistrement de données (D1) contenant l'information et au moins un paramètre de gaz.

10. Méthode selon la revendication 9, selon laquelle l'information contenue dans le moyen d'identification (2) est lue sans contact, de préférence optiquement ou électromagnétiquement.

11. Méthode selon l'une des revendications 9 ou 10, selon laquelle l'information contenue dans le moyen d'identification (2) est lue par un contact mécanique, de préférence au moyen de capteurs tactiles.

12. Méthode selon l'une des revendications 9 à 11, selon laquelle un transpondeur RFID, un code-barres ou un relief détectable mécaniquement est utilisé comme moyen d'identification (2).

13. Méthode selon l'une des revendications 9 à 12, selon laquelle l'une ou plusieurs des informations suivantes du système de contrôle de gaz sont lues à partir du moyen d'identification (2): emplacement de montage de la prise de gaz, pression de consigne maximale et/ou minimale.

14. Méthode selon l'une des revendications 9 à 13, selon laquelle l'information est saisie au moyen d'un lecteur de code-barres, d'un appareil pour la réception d'informations enregistrées dans un transpondeur RFID ou d'un dispositif de balayage pour balayer un relief.

15. Méthode selon l'une des revendications 9 à 14, selon laquelle le dispositif de saisie (4) est monté dans la zone d'un raccord du tuyau de gaz (3), de sorte que l'information soit lue seulement en cas d'un raccordement du raccord à la prise de gaz.

16. Méthode selon l'une des revendications 9 à 15, selon laquelle au moins l'un des paramètres de gaz suivants est mesuré au moyen du dispositif de mesure (7): pression, de préférence pression statique et/ou pression dynamique, température, débit volumique, débit massique, point de rosée, teneur en humidité, teneur en O₂, teneur en CO₂, teneur en N₂O.

17. Méthode selon l'une des revendications 9 à 16, selon laquelle les paramètres de gaz mesurés sont affichés à l'aide d'un moyen d'affichage, de préférence d'un écran plat.

18. Méthode selon l'une des revendications 9 à 17, selon laquelle les paramètres de gaz mesurés sont mémorisés dans un moyen (5a) servant à l'enregistrement.

19. Méthode selon l'une des revendications 9 à 18, selon laquelle l'enregistrement de données (D1) est transmis via une interface (11) à un dispositif de traitement des données.
